(19) Europäisches Patentamt
European Patent Office
Office européen des brevets

(11) Publication number: **0 190 221**
**B1**

(12) # EUROPEAN PATENT SPECIFICATION

(45) Date of publication of the patent specification: **02.11.88**

(51) Int. Cl.⁴: **C 07 C 33/34**, C 07 C 33/36

(21) Application number: **85903759.0**

(22) Date of filling: **17.07.85**

(86) International application number: **PCT/US 85/01355**

(87) International publication number: **WO 86/00886 (13.02.86 Gazette 86/04)**

(54) **PROCESS FOR HYDROXYMETHYLATION.**

(30) Priority: **26.07.84 US 634912**

(43) Date of publication of application: **13.08.86 Bulletin 86/33**

(45) Publication of the grant of the patent: **02.11.88 Bulletin 88/44**

(84) Designated Contracting States: **AT BE CH DE FR GB IT LI NL**

(56) References cited:
**US-A-2 768 994**
**US-A-2 828 286**
**US-A-2 841 570**
**US-A-2 844 561**
**US-A-2 848 437**
**US-A-2 848 500**
**US-A-2 873 297**
**US-A-2 957 036**
**US-A-2 964 500**
**US-A-4 024 163**
**US-A-4 087 468**
**US-A-4 089 908**
**US-A-4 130 657**
**US-A-4 214 004**
**US-A-4 329 518**
**US-A-4 402 973**

**No relevant documents have been disclosed.**
**Recueil, Volume 84, published 1965 Schaap et al.**

(73) Proprietor: **FMC Corporation, 2000 Market Street, Philadelphia Pennsylvania 19103 (US)**

(72) Inventor: **DELMAR, Eric, George, 116 Featherbed Lane, Hopewell, NJ 08525 (US)**
Inventor: **KWIATKOWSKI, Charles, Thomas, 11- 07 Pheasant Hollow Drive, Plainsboro, NJ 08536 (US)**

(74) Representative: **Mongrédien, André, c/o SOCIETE DE PROTECTION DES INVENTIONS 25, rue de Ponthieu, F-75008 Paris (FR)**

(56) References cited: (continuation)
**See pages 1200-3**
**Encyclopedia of Chemical Technology, 3rd ed, published 1978, Vol. 1 Kirk Othmer, See pages 112-113**
**Formaldehyde, 3rd Ed. American Chemical Society Monograph Series, published 1964, Walked See Chapter 7**
**Reagents For Organic Synthesi, Publisched 1967, Fieser et al. See pages 415-424**
**Organic Syntheses, Coll. Vol. 1, published 1941, Gilman et al. See page 188 Cyclohexylcarbinol**

**EP 0 190 221 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension).

LIBER, STOCKHOLM 1988

**Description**

This invention is in the field of chemical processes and relates to a method for hydroxymethylating organic substrates to produce hydroxymethyl derivatives thereof, sometimes referred to herein as "methylol" compounds or "carbinols". In particular, the present invention pertains to a process for producing methylol compounds by the reaction of an organometallic compound with formaldehyde produced in situ from a high molecular weight linear formaldehyde homopolymer.

Methylol compounds of various structures abound in the chemical literature. Many such compounds are useful end-products in themselves, while others are useful as intermediates in the preparation of various useful end-products. For example, certain substituted-phenylmethyl alcohols, such as the various biphenylmethanols disclosed in U.S. Patent Nos. 4 130 657; 4 214 004; 4 329 518; and 4 402 973 are useful intermediates for insecticidal pyrethroid esters.

A number of methods for producing methylol compounds are available to the chemical practitioner. For compounds having one or more active carbon-bonded protons, capable of being removed by a base, and for halogen-containing compounds from which Grignard reagents can be produced, the usual method for introducing a hydroxymethyl group into the molecule, in place of the active hydrogen or the halogen, is by reaction of the Grignard reagent or a metal salt of the organic substrate with formaldehyde. This general method is illustrated in the following chemical equation in which R is an organic radical such as a hydrocarbyl radical and M is a metal or metal-halide:

$$R\text{-}M + CH_2O \rightarrow RCH_2OH.$$

Where the carbon-bonded active proton of the organic substrate is highly acidic, for example, the α protons of diethyl malonate, the hydroxymethylation reaction may be conducted simply and conveniently in water in the presence of a base such as potassium bicarbonate, the formaldehyde reactant being commercially available as an aqueous solution.

However, for Grignard or similar hydroxymethylation reactions where the organometallic substrate is subject to decomposition by water, generally where the $pK_b$ value for the organo-metallic material is lower than about -5, the use of aqueous formaldehyde is contraindicated. In such cases precautions must be taken to exclude water from the reaction mixture to preserve the integrity of the organometallic substrate for reaction with the formaldehyde. Generally, the formaldehyde reactant is generated from the solid polymer paraformaldehyde which depolymerizes or "unzips" to produce formaldehyde vapors. The depolymerization is facilitated by heat and is usually accomplished either in situ on heating the reaction mixture containing the paraformaldehyde or by pyrolyzing the paraformaldehyde in a separate vessel and conducting the vapors so produced into the hydroxymethylation reaction vessel.

The use of paraformaldehyde directly as a source of dry formaldehyde, however, has not been entirely satisfactory inasmuch as paraformaldehyde itself is not anhydrous, but contains a certain amount of free water in addition to the chemically combined water that is part of the paraformaldehyde molecule itself and is released in the unzipping process. The Kirk-Othmer Encyclopedia of Chemical Technology, 3rd ed; John Wiley & Sons, New York 1978; Vol. 11; page 247 states that commercial paraformaldehyde (average molecular weight of about 600) in powder form contains up to 5 % free and combined water and in flake form, up to 9 %. Thus, use of commercial paraformaldehyde as such in a Grignard or similar organometallic hydroxymethylation reaction as a source of the requisite formaldehyde may result in the inclusion in the reaction mixture of up to 5 % or more of water and the destruction of an equivalent amount of the organometallic reagent.

While formaldehyde vapors may be generated by the pyrolysis of paraformaldehyde outside the hydroxymethylation reaction vessel and subjected to appropriate drying conditions prior to bringing it into contact with the organometallic substrate, such a method adds an additional step to the hydroxymethylation process and may unduly subject the worker and other nearby personnel to the possibility of coming into contact with formaldehyde vapor or dust which is highly irritating, particularly to the eyes and respiratory tract, and may cause skin sensitization or dermatitis.

The present invention represents an improvement over the prior art method of hydroxymethylation in that an inherently drier source of formaldehyde than previously used is employed in the present process thereby reducing aqueous decomposition of the organometallic substrate and allowing for increased yields of the desired carbinol products. Furthermore, the present high molecular weight linear formaldehyde homopolymer, like paraformaldehyde, may be added directly to the reaction mixture allowing the formaldehyde being formed to be consumed in the desired reaction concomitantly with its formation, thereby reducing the risk to personnel of exposure to formaldehyde fumes.

In the present invention, the requisite formaldehyde is generated in situ from and by the use of a high molecular weight linear formaldehyde homopolymer. The high molecular weight formaldehyde homopolymers useful in the present invention are polyoxymethylene compounds having a molecular weight of at least about 10,000. The polymers are composed of repeating oxymethylene units which form a linear acetal chain $\sim O\text{-}CH_2\text{-}O\text{-}CH_2 \sim$ and are terminated at each end by a hydroxy group or by a labile end-capping group such as an ester group, or are terminated at one end by a non-labile end-capping group such as an ether group and at the other end by a labile end-capping group. Polymers that are capped at only one end, by a labile or a non-labile end-capping group, are also useful in the present process and are within the scope of the present invention.

Labile end-capping groups are groups that are removable under the conditions of the hydroxymethylation process, whereas non-labile groups are not removable under such conditions. In order for a formaldehyde homopolymer of appropriate molecular weight to be capable of being depolymerized or unzipped to produce formaldehyde under typical hydroxymethylation process conditions and, therefore, to be suitable for use in the present process, it is necessary that at least one terminal of the formaldehyde polymer be occupied by a hydroxy group or a labile end-capping group.

It is well known in the art of polymer chemistry, as disclosed in U.S. Patent No. 2 964 500, that polymer compositions, by virtue of limitations inherent in the methods known and used in the art for producing them, are not homogeneous but are mixtures of molecules of various molecular weights generally concentrated within a narrow range. Polymer compositions are also inexact with respect to end-caps. For example, in a composition of polymers having an ether group or an ester group at one end and a hydroxy group at the other, a small portion of the composition will likely consist of polymers capped at both ends as well as completely uncapped polymers. Therefore, it will be understood that molecular weights recited herein for formaldehyde polymers useful in the present process are average molecular weights and that some small portion of the polymer composition may differ from the major portion of the composition. The limitations described herein to define the formaldehyde polymers suitable for use in the present process are fully met if met by the polymers which constitute the major portion of a polymer composition.

The formaldehyde homopolymers useful herein may have an average molecular weight up to about 1,000,000 or higher. The average molecular weight will usually be in the range of 10,000 to 200,000, preferably in the range of 15,000 to 150,000, and more preferable in the range of 20,000 to 100,000. Those materials having an average molecular weight in the range of about 25,000 to 75,000 are especially preferred. Included among this last group of preferred polymers are polyoxymethylene diacetates such as the Delrin brand of acetal homopolymer resins sold by E.I. du Pont de Nemours & Co., Inc., Wilmington, Delaware. Polyoxymethylene diacetates having an average molecular weight of about 50,000, such as Delrin 500, are particularly desirable. Suitable formaldehyde polymers are also described in various patents and literature in the art. Both the polyoxymethylene glycol starting materials and diacetate products described in U.S. Patent 2 964 500, as well as the polyoxymethylene products described in U.S. Patents 2 768 994; 2 828 286; 2 841 570; and 2 848 437, all of which patents are incorporated herein by reference, are suitable for use in the present process. The polyoxymethylene products disclosed in U.S. Patent No. 2 844 561, incorporated herein by reference, and which have incorporated in their structures a small amount of an amine polymerization initiator are also generally useful in the present invention but are not preferred. Other references disclosing the preparation and properties of high molecular weight formaldehyde homopolymers, including materials useful herein, are "The Kirk-Othmer Encyclopedia of Chemical Technology", 3rd ed.; John Wiley & Sons, New York; 1978; Vol. 1; pages 112-123 and "Formaldehyde"; ACS Monograph Series; J. Frederic Walker, Ed.; 3rd ed; Reinhold Publishing Corp., New York; 1964; Chapter 7.

The high molecular weight linear formaldehyde polymers useful in the present invention are compounds of the formula

$$R^1O\text{-}(CH_2O)_n\text{-}R^2 \qquad \text{I}$$

wherein $R^1$ and $R^2$ are independently hydrogen or an end-cap group that is removable under the conditions employed in the hydroxymethylation process. One of $R^1$ and $R^2$, but not both, may also be a group that is not removable under the hydroxymethylation conditions. Thus, either uncapped or certain end-capped polymers may be used.

Uncapped formaldehyde polymers and polymers capped at only one terminal may depolymerize on standing to release formaldehyde. As with paraformaldehyde, depolymerization of the higher molecular weight uncapped or partially capped polymers useful herein is facilitated by heat.

$$HOCH_2CH_2O(CH_2O)_nH \xrightarrow{\Delta} n\,CH_2O + HOCH_2CH_2OH$$

$$HO(CH_2O)_nH \xrightarrow{\Delta} n\,CH_2O + H_2O$$

Formaldehyde polymers end-capped at both terminals offer an advantage over the corresponding uncapped materials in that they are storage-stable and will not depolymerize to release formaldehyde fumes until removal of at least one of the end-caps.

End-caps that are not removable under hydroxymethylation process conditions, and which may be present at one terminal but not both, include typical ether forming groups such as alkyl, aryl, and arylalkyl radicals and substituted derivatives thereof, for example, methyl, methoxyethyl, hydroxyethyl, hydroxypolyethoxyethyl, methoxypolyethoxyethyl, polyethoxyethyl, phenyl, and benzyl.

End-caps that are removable under hydroxymethylation process conditions include acyl and similar ester forming groups that are reactive themselves toward the organometallic substrate and are ultimately eliminated from the polymer leaving the previously capped end of the molecule uncapped and free to depolymerize. Typical removable end-caps are radicals such as $-CQ-R^3$ or $-P(Q)R^4R^5$ wherein $R^3$, $R^4$ and $R^5$ independently are

optionally substituted alkyl, alkoxy, alkylthio, aryl, aryloxy, arylthio, arylalkyl, arylalkoxy, or arylalkylthio and Q is O or S. Preferably, $R^3$, $R^4$, and $R^5$ are independently selected from alkyl, aryl, and arylalkyl each of which may be substituted. A preferred end-cap ($R^1$ or $R^2$ or both) is the radical $-CO-R^3$, especially $-CO-CH_3$. In a preferred embodiment of the invention, the formaldehyde polymer is a polyoxymethylene diacetate ($R^1 = R^2 = -CO-CH_3$) having an average molecular weight in the range of about 25,000 to 75,000, particularly the material having an average molecular weight of about 50,000. Use of the corresponding glycol ($R^1 = R^2 =$ hydrogen) also represents a preferred embodiment of the invention. Examples of removable end-caps are radicals such as $-CO-CH_3$, $-CO-CH(CH_3)_2$, $-CO-CH(CH_2)_4CH_2$, $-CO-C_6H_5$, $-CO-CH_2C_6H_5$, $-CS-CH_3$, $-CO_2CH_3$, $-CS_2CH_3$, $-CO-CF_3$, $-CO-SCH_3$, $-CO_2C_6H_5$, $-CO_2CH_2C_6H_5$, $-P(O)(CH_3)_2$, $-P(S)(CH_3)(SC_2H_5)$, $-P(O)(OC_2H_5)_2$, $-P(S)(CH_3)(OC_2H_5)$, and $-P(O)(C_6H_5)(OC_2H_5)$.

Any alkyl or aryl substituent or portion of a substituent herein may be substituted, for example, with halogen such as fluorine, chlorine, or bromine, cyano, nitro, hydroxy, alkoxy, alkylthio, or the like. Additionally, any aryl group may be substituted with alkyl. Any alkyl may be a straight chain, branched chain, or cyclic radical, frequently a lower alkyl of 1 to 8 (preferably 1 to 4) carbon atoms. Any aryl group herein may be a hydrocarbyl radical such as phenyl or a heteroaryl radical, for example, furyl, thienyl, pyridyl, pyrimidyl, oxazolyl, pyrrolyl, isoxazolyl, thiazolyl, or isothiazolyl. Halogenated alkyl or aryl groups may have one or more same or different halogen atoms.

The high molecular weight formaldehyde polymers described above are useful as a source of formaldehyde reactant in hydroxymethylation processes in which an organometallic substrate is reacted with formaldehyde to produce a methylol compound. Since the present polymers represent a source of substantially anhydrous formaldehyde, they are especially useful in those instances where the organometallic substrate is sensitive to water.

The present high molecular weight formaldehyde polymers are pulverulent solids and are advantageously employed in the present process in the form of a fine powder. It is preferred, for increased reaction rates, that the particle size be sufficiently small for the powder to pass through a 300 µm (50 mesh U.S.) sieve. It is desirable, therefore, to pulverize or grind coarser grade material to obtain a fine particle size.

The organometallic compound is any compound such as phenylmagnesium chloride or phenyllithium which reacts with formaldehyde under anhydrous hydroxymethylation conditions to produce a carbinol. For example, the organometallic compound may be an organomagnesium halide compound or an organozinc halide compound in which the halide is a chlorine, bromine, or iodine atom, an organolithium compound, an organosodium compound, an organopotassium compound, or the like. Frequently, the organometallic compound will be an organomagnesium halide compound. The organic moiety will generally be strongly associated with the metal moiety via an ionic carbon-to-metal bond, although the degree of association will generally vary with the polarity of the solvent used in the hydroxymethylation process. The organic moiety may be aromatic or non-aromatic, it may be a hydrocarbyl radical or contain one or more heteroatoms, and it may or may not have one or more points of unsaturation as, for example, an alkynyl radical $R-C{\equiv}C-$.

In a preferred embodiment of the present invention, the organometallic substrate is an arylmagnesium halide, for example, a phenylmagnesium halide. Phenylmagnesium halides of particular interest include those of the formula

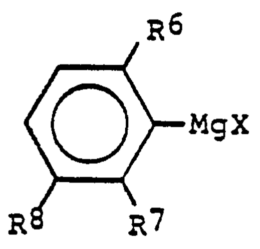

II

in which X is chlorine, bromine, or iodine and $R^6$ $R^7$, and $R^8$ are independently hydrogen, halogen such as chlorine, or lower alkyl. $R^8$ may also be phenyl which may be substituted with halogen or lower alkyl. The carbinol products of this embodiment are useful intermediates for insecticidal pyrethroid esters, especially where $R^6$ is hydrogen or methyl, more especially hydrogen, $R^7$ is methyl, and $R^8$ is chlorine or, particularly phenyl.

The present process is illustrated for a preferred embodiment in the following chemical equation:

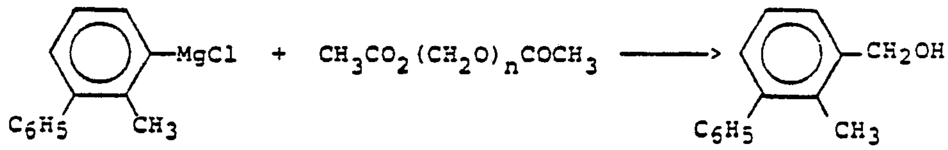


IIa Ia III

Reaction of the organometallic compound with the polyoxymethylene diacetate results in the removal of one or both acetyl groups from the formaldehyde polymer, and the resulting polymer product then depolymerizes to generate formaldehyde which reacts with additional organometallic compound to produce the carbinol product.

The present process may be conducted under any reaction conditions that would be acceptable in a hydroxymethylation process involving the reaction of the same organometallic compound with formaldehyde in which the formaldehyde is generated in situ from paraformaldehyde. At least a stoichiometric amount of the formaldehyde polymer, calculated on the basis of the amount of polymerized formaldehyde present, should be employed.

In general, the organometallic compound (produced in situ or separately) is brought into contact with the high molecular weight formaldehyde polymer under substantially anhydrous conditions in the presence of a substantially inert solvent under a substantially inert atmosphere. As with similar organometallic reactions, care should be taken to exclude moisture, oxygen, and carbon dioxide, which may react with the organometallic compound, by using an inert atmosphere such as nitrogen or helium.

The reaction may by conducted under standard temperature conditions for similar reactions, generally at a temperature in the range of about 25° to 150°C, advantageously at an elevated temperature such as a temperature of about 60°, preferably up to about 100°C. Frequently, the upper temperature limit will be the boiling point of the solvent or a temperature just below the melting point of the formaldehyde polymer. Preferably, the insoluble solid formaldehyde polymer is maintained as a fine powder in the reaction mixture to facilitate the generation of formaldehyde. If allowed to melt, the polymer may agglomerate into a gummy mass which may hinder the reaction.

Any of the solvents typically used in similar organometallic reactions, such as ethers or hydrocarbon solvents, may be suitably used in the present process. Some organometallic compounds, particularly organosodium compounds, are known in the art to be highly reactive toward diethyl ether, and in such cases, as in the prior art processes, a different solvent should be selected. Tetrahydrofuran is a preferred solvent.

As in the prior art hydroxymethylation processes involving the reaction of an organometallic compound with formaldehyde and conducted under anhydrous conditions, the carbinols are produced in the present process in the form of complex salts from which the desired carbinols are freed by acid hydrolysis. This involves simply contacting the salt with water and adjusting the pH by the addition of sufficient acid or acidic material to free the carbinol from the salt. Generally, the reaction mixture is poured over ice and the mixture is made acidic by the addition of an acid such as hydrochloric acid, usually concentrated hydrochloric acid. Where the desired carbinol product is sensitive to strong acids, the hydrolysis will usually be carried out by the addition of an acidic material such as ammonium chloride, generally as a saturated aqueous solution.

The present invention is further illustrated in the following example.

**Example**

Synthesis of (2-methyl[1,1'-biphenyl]-3-yl)-methanol

A. Preparation of (2-methyl[1,1'-biphenyl]-3-yl)magnesium chloride

Under a nitrogen atmosphere, 101.3 g (0.50 mole) of 3-chloro-2-methylbiphenyl (92 % purity) in 75 g of dry tetrahydrofuran was added dropwise over two hours with stirring to a gently refluxing mixture of 13.1 g (0.54 mole) of fresh magnesium turnings, the heel from a previous run containing 0.21 mole of unreacted magnesium, and 75 g of dry tetrahydrofuran in a dry reaction vessel. Upon complete addition, the reaction mixture was heated at reflux for five additional hours. Gas chromatographic analysis of a quenched sample of the reaction mixture showed 1.2 % of unreacted 5-chloro-2-methylbiphenyl and 92.4 % of 2-methylbiphenyl. The reaction mixture was allowed to cool overnight.

B. Preparation of (2-methyl[1,1'-biphenyl]-3-yl)methanol

Under a nitrogen atmosphere, the reaction mixture from above was decanted into a clean, dry flask, leaving the unreacted magnesium turnings in the first flask. This solution, which contains (2-methyl[1,1'-biphenyl]-3-yl)magnesium chloride, was heated to reflux, and 16.5 g (0.55 mole based on formaldehyde) of polyoxymethylene diacetate (avg. mol. wgt. approx. 50,000, available commercially from E.I. du pont de

Nemours & Co., Inc., Wilmington, Delaware under the trade name Delrin 500 acetal homopolymer resin), milled to about 80 mesh, was added with stirring over three hours. Refluxing was continued for four additional hours after which the reaction mixture was allowed to cool and stand overnight. The mixture was then heated to 53°C and was poured into a mixture of 100 g of ice, 52 g of concentrated hydrochloric acid, and 200 g of a mixture containing 95 % n-octane and 5 % toluene (wt/wt). This mixture was filtered and the organic phase separated. The organic solvents were distilled off, boiling range 65 - 93°C. During a four hour period the solution was cooled with stirring to approximately 5°C and was then filtered. The filter cake was washed with 100 ml of cold n-octane and air-dried for 0.5 hour. The filter cake was broken up and dried under a vacuum at 55°C for four hours, yielding 78.9 g of (2-methyl[1,1'-biphenyl]-3-yl)-methanol (89.9 % purity), a yield of 70.8 % of theory.

## Claims

1. A method for producing a methylol compound by the reaction of an organometallic compound with formaldehyde, characterized by generating the formaldehyde in situ from and by the use of a linear formaldehyde homopolymer having an average molecular weight of at least 10,000.

2. The method of claim 1 characterized in that the formaldehyde polymer is terminated at each end by a hydroxy group or by a labile end-capping group, or is terminated at one end by a non-labile end-capping group and at the other end by a labile end-capping group or is terminated at one end by a hydroxy group and at the other end by a labile or a non-labile end-capping group.

3. The method of claim 2 characterized in that the formaldehyde polymer has an average molecular weight in the range of 10,000 to 200,000.

4. The method of claim 3 characterized in that the labile end-capping group is a labile ester group and the non-labile end-capping group is an ether group.

5. The method of claim 4 characterized in that the formaldehyde polymer is a polymer of the formula

$$R^1O\text{-}(CH_2O)_n\text{-}R^2$$

in which $R^1$ and $R^2$ are independently hydrogen, a non-labile radical selected from the group consisting of alkyl, aryl, arylalkyl, and substituted derivatives thereof, or a labile radical of the formula $-CQ-R^3$ or $-P(Q)R^4R^5$ in which $R^3$, $R^4$, and $R^5$ are independently selected from alkyl, alkoxy, alkylthio, aryl, aryloxy, arylthio, arylalkyl, arylalkoxy, arylalkylthio, and substituted derivatives thereof and Q is oxygen or sulfur, with the proviso that at least one of $R^1$ and $R^2$ is hydrogen, $-CQ-R^3$, or $-P(Q)R^4R^5$.

6. The method of claim 5 characterized in that for $R^1$, $R^2$, $R^3$, $R^4$, and $R^5$, any alkyl group or the alkyl portion of any group is lower alkyl or a substituted lower alkyl and any aryl group or the aryl portion of any group is phenyl or a substituted phenyl.

7. The method of claim 6 characterized in that $R^1$ and $R^2$ are independently selected from hydrogen and a labile radical $-CO-R^3$ in which $R^3$ is lower alkyl.

8. The method of claim 7 characterized in that each of $R^1$ and $R^2$ is hydrogen.

9. The method of claim 7 characterized in that each of $R^1$ and $R^2$ is $-CO-CH_3$.

10. The method of claim 8 or 9 characterized in that the organometallic compound is selected from the group consisting of an organomagnesium halide compound, an organozinc halide compound, an organolithium compound, an organosodium compound, and an organopotassium compound, wherein halide is a chlorine, bromine, or iodine atom.

11. The method of claim 10 characterized in that the organometallic compound is an organomagnesium halide compound.

12. The method of claim 11 characterized in that the organomagnesium halide compound is an arylmagnesium halide compound.

13. The method of claim 12 characterized in that the arylmagnesium halide compound is a phenylmagnesium halide compound and the methylol product is a phenylmethyl alcohol.

14. The method of claim 13 characterized in that the phenylmagnesium halide compound is a compound of the formula

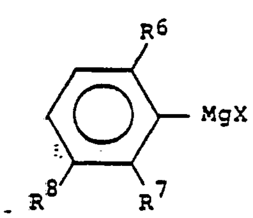

wherein X is chlorine, bromine, or iodine, $R^6$ and $R^7$ are independently hydrogen, halogen, or lower alkyl, and $R^8$ is hydrogen, halogen, lower alkyl, or phenyl which is unsubstituted or substituted with halogen or lower

alkyl, and the methylol product is a phenylmethyl alcohol of the formula

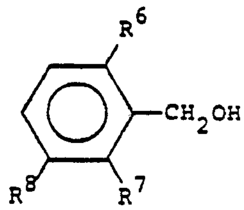

in which $R^6$, $R^7$, and $R^8$ are defined as above.

15. The method of claim 14 characterized in that $R^6$ is hydrogen or methyl, $R^7$ is methyl, and $R^8$ is chlorine or phenyl, with the proviso that when $R^8$ is chlorine $R^6$ is hydrogen.

16. The method of claim 15 characterized in that $R^6$ is hydrogen.

17. The method of claim 16 characterized in that $R^8$ is phenyl.

18. The method of claim 17 which is conducted under substantially anhydrous conditions, in the presence of a substantially inert atmosphere, at a temperature of 25°C up to a temperature below the melting point of the formaldehyde polymer.

19. The process of claim 18 characterized in that the temperature is in the range of 60° to 100°C.

20. The process of claim 19 characterized in that the formaldehyde polymer has an average molecular weight in the range of 15,000 to 150,000.

21. The method of claim 20 characterized in that the average molecular weight of the formaldehyde polymer is in the range of 20,000 to 100,000.

22. The method of claim 21 characterized in that the average molecular weight of the formaldehyde polymer is in the range of 25,000 to 75,000.

**Patentansprüche**

1. Verfahren zur Herstellung einer Methylolverbindung durch Umsetzen einer Organometallverbindung mit Formaldehyd, gekennzeichnet durch Bildung des Formaldehyds in situ aus und durch die Verwendung eines linearen Formaldehydhomopolymers mit einem mittleren Molgewicht von mindestens 10 000.

2. Verfahren nach Anspruch 1, dadurch gekennzeichnet, daß das Formaldehydpolymer an jedem Ende von einer Hydroxygruppe oder einer labilen Endschutzgruppe terminiert ist oder an einem Ende von einer nicht-labilen Endschutzgruppe und am anderen Ende von einer labilen Endschutzgruppe terminiert ist oder an einem Ende von einer Hydroxygruppe und am anderen Ende von einer labilen oder nicht-labilen Endschutzgruppe terminiert ist.

3. Verfahren nach Anspruch 2, dadurch gekennzeichnet, daß das Formaldehydpolymer ein mittleres Molgewicht im Bereich von 10 000 bis 200 000 aufweist.

4. Verfahren nach Anspruch 3, dadurch gekennzeichnet, daß die labile Endschutzgruppe eine labile Estergruppe und die nichtlabile Endschutzgruppe eine Äthergruppe ist.

5. Verfahren nach Anspruch 4, dadurch gekennzeichnet, daß das Formaldehydpolymer ein Polymer der Formel

$$R^1O\text{-}(CH_2O)_n\text{-}R^2$$

ist, worin $R^1$ und $R^2$ unabhängig voneinander Wasserstoff, ein nicht-labiler Rest ausgewählt aus der Gruppe bestehend aus Alkyl, Aryl, Arylalkyl und substituierten Derivaten hievon, oder ein labiler Rest der Formel $-CQ-R^3$ oder $-P(Q)R^4R^5$ sind, wobei $R^3$, $R^4$ und $R^5$ unabhängig voneinander ausgewählt sind aus Alkyl, Alkoxy, Alkylthio, Aryl, Aryloxy, Arylthio, Arylalkyl, Arylalkoxy, Arylalkylthio und substituierten Derivaten hievon, und Q Sauerstoff oder Schwefel bedeutet, mit der Maßgabe, daß mindestens eines von $R^1$ und $R^2$ Wasserstoff, $-CQ-R^3$ oder $P(Q)R^4R^5$ ist.

6. Verfahren nach Anspruch 5, dadurch gekennzeichnet, daß für $R^1$, $R^2$, $R^3$, $R^4$ und $R^5$ jede Alkylgruppe oder der Alkylteil irgendeiner Gruppe nied.Alkyl oder substituiertes nied.Alkyl ist und jede Arylgruppe oder der Arylteil irgendeiner Gruppe Phenyl oder substituiertes Phenyl ist.

7. Verfahren nach Anspruch 6, dadurch gekennzeichnet, daß $R^1$ und $R^2$ unabhängig voneinander ausgewählt sind aus Wasserstoff und einem labilen Rest $-CO-R^3$, wobei $R^3$ nied.Alkyl ist.

8. Verfahren nach Anspruch 7, dadurch gekennzeichnet, daß $R^1$ und $R^2$ jeweils Wasserstoff sind.

9. Verfahren nach Anspruch 7, dadurch gekennzeichnet, daß $R^1$ und $R^2$ jeweils $-CO-CH_3$ sind.

10. Verfahren nach Anspruch 8 oder 9, dadurch gekennzeichnet, daß die Organometallverbindung ausgewählt ist aus der Gruppe bestehend aus einer Organomagnesiumhalogenidverbindung, einer Organozinkhalogenidverbindung, einer Organolithiumverbindung, einer Organonatriumverbindung und einer Organokaliumverbindung, wobei Halogenid ein Chlor-, Brom- oder Jodatom ist.

11. Verfahren nach Anspruch 10, dadurch gekennzeichnet, daß die Organometallverbindung eine Organomagnesiumhalogenidverbindung ist.

12. Verfahren nach Anspruch 11, dadurch gekennzeichnet, daß die Organomagnesiumhalogenidverbindung eine Arylmagnesiumhalogenidverbindung ist.

13. Verfahren nach Anspruch 12, dadurch gekennzeichnet, daß die Arylmagnesiumhalogenidverbindung eine Phenylmagnesiumhalogenidverbindung ist und das Methylolprodukt ein Phenylmethylalkohol ist.

14. Verfahren nach Anspruch 13, dadurch gekennzeichnet, daß die Phenylmagnesiumhalogenidverbindung eine Verbindung der Formel

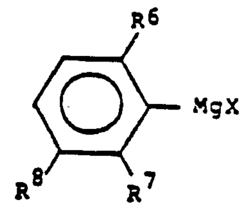

ist, worin X Chlor, Brom oder Jod bedeutet, $R^6$ und $R^7$ unabhängig voneinander jeweils Wasserstoff, Halogen oder nied.Alkyl darstellen und $R^8$ Wasserstoff, Halogen, nied.Alkyl oder Phenyl, das unsubstituiert oder mit Halogen oder nied.Alkyl substituiert ist, bedeutet, und das Methylolprodukt ein Phenylmethylalkohol der Formel

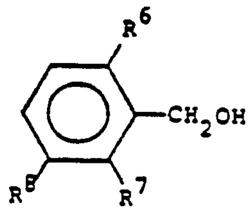

ist worin $R^6$, $R^7$ und $R^8$ wie oben definiert sind.

15. Verfahren nach Anspruch 14, dadurch gekennzeichnet, daß $R^6$ Wasserstoff oder Methyl bedeutet, $R^7$ Methyl ist und $R^8$ Chlor oder Phenyl darstellt, mit der Maßgabe, daß, wenn $R^8$ Chlor ist, $R^6$ Wasserstoff bedeutet.

16. Verfahren nach Anspruch 15, dadurch gekennzeichnet, daß $R^6$ Wasserstoff ist.

17. Verfahren nach Anspruch 16, dadurch gekennzeichnet, daß $R^8$ Phenyl ist.

18. Verfahren nach Anspruch 17, das unter praktisch wasserfreien Bedingungen in Anwesenheit einer im wesentlichen inerten Atmosphäre bei einer Temperatur von 25°C bis zu einer Temperatur unterhalb des Schmelzpunktes des Formaldehydpolymers durchgeführt wird.

19. Verfahren nach Anspruch 18, dadurch gekennzeichnet, daß die Temperatur im Bereich von 60 bis 100°C liegt.

20. Verfahren nach Anspruch 19, dadurch gekennzeichnet, daß das Formaldehydpolymer ein mittleres Molgewicht im Bereich von 15 000 bis 150 000 aufweist.

21. Verfahren nach Anspruch 20, dadurch gekennzeichnet, daß das mittlere Molgewicht des Formaldehydpolymers im Bereich von 20 000 bis 100 000 liegt.

22. Verfahren nach Anspruch 21, dadurch gekennzeichnet, daß das mittlere Molgewicht des Formaldehydpolymers im Bereich von 25 000 bis 75 000 liegt.

**Revendications**

1. Procédé de préparation d'un composé méthylolé par réaction d'un composé organométallique avec du formaldéhyde, caractérisé en ce qu'on produit le formaldéhyde in situ et en ce que l'on utilise un homopolymère du formaldéhyde linéaire ayant une masse moléculaire moyenne d'au moins 10 000.

2. Procédé de la revendication 1, caractérisé en ce que le polymère du formaldéhyde est terminé à chaque extrémité par un groupe hydroxy ou par un groupe de coiffage d'extrémité labile, ou est terminé à une extrémité par un groupe de coiffage d'extrémité non labile et à l'autre extrémité par un groupe de coiffage d'extrémité labile, ou est terminé à une extrémité par un groupe hydroxy et à l'autre extrémité par un groupe de coiffage d'extrémité labile ou non labile.

3. Procédé de la revendication 2, caractérisé en ce que le polymère du formaldéhyde a une masse moléculaire moyenne dans l'intervalle de 10 000 à 200 000.

4. Procédé de la revendication 3, caractérisé en ce que le groupe de coiffage d'extrémité labile est un groupe

ester labile et en ce que le groupe de coiffage d'extrémité non labile est un groupe éther.

5. Procédé de la revendication 4, caractérisé en ce que le polymère du formaldéhyde est un polymère répondant à la formule:

$$R^1O\text{-}(CH_2O)_n\text{-}R^2$$

dans laquelle $R^1$ et $R^2$ sont indépendamment un atome d'hydrogène, un radical non labile choisi dans le groupe constitué des radicaux alkyle, aryle, arylalkyle et de leurs dérivés substitués, ou un radical labile répondant à la formule:

$$\text{-CQ-}R^3 \text{ ou -P(Q)}R^4R^5$$

dans laquelle $R^3$, $R^4$ et $R^5$ sont choisis indépendamment parmi les radicaux alkyle, alcoxy, alkylthio, aryle, aryloxy, arylthio, arylalkyle, arylalcoxy, arylalkylthio, et leurs dérivés substitués et Q est l'oxygène ou le soufre, sous réserve qu'au moins un des symboles $R^1$ et $R^2$ soit un atome d'hydrogène, -CQ-$R^3$, ou -P(Q)$R^4R^5$.

6. Procédé de la revendication 5, caractérisé en ce que pour $R^1$, $R^2$, $R^3$, $R^4$, et $R^5$, n'importe quel groupe alkyle ou la partie alkyle de n'importe quel groupe est un radical alkyle inférieur ou un radical alkyle inférieur substitué et n'importe quel groupe aryle ou la partie aryle de n'importe quel groupe est un groupe phényle ou un groupe phényle substitué.

7. Procédé de la revendication 6, caractérisé en ce que $R^1$ et $R^2$ sont choisis indépendamment parmi un atome d'hydrogène et un radical labile -CO-$R^3$ dans lequel $R^3$ est un radical alkyle inférieur.

8. Procédé de la revendication 7, caractérisé en ce que chacun des symboles $R^1$ et $R^2$ est un atome d'hydrogène.

9. Procédé de la revendication 7, caractérisé en ce que chacun des symboles $R^1$ et $R^2$ est -CO-$CH_3$.

10. Procédé de la revendication 8 ou 9, caractérisé en ce que le composé organométallique est choisi dans le groupe constitué d'un halogénure d'organomagnésium, d'un halogénure d'organozinc, d'un composé d'organolithium, d'un composé d'organosodium, et d'un composé d'organopotassium, dans lequel l'halogénure est un atome de chlore, de brome ou d'iode.

11. Procédé de la revendication 10, caractérisé en ce que le composé organométallique est un halogénure d'organomagnésium.

12. Procédé de la revendication 11, caractérisé en ce que l'halogénure d'organomagnésium est un halogénure d'arylmagnésium.

13. Procédé de la revendication 12, caractérisé en ce que l'halogénure d'arylmagnésium est un halogénure de phénylmagnésium et en ce que le produit méthylolé est un alcool phénylméthylique.

14. Procédé de la revendication 13, caractérisé en ce que l'halogénure de phénylmagnésium est un composé répondant à la formule:

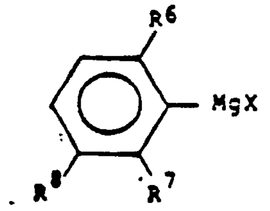

dans laquelle X est un atome de chlore, de brome, ou d'iode, $R^6$ et $R^7$ sont indépendamment un atome d'hydrogène, un atome d'halogène ou un groupe alkyle inférieur, et $R^8$ est un atome d'hydrogène, un atome d'halogène, un groupe alkyle inférieur ou un groupe phényle qui est non substitué ou substitué par un atome d'halogène ou par un groupe alkyle inférieur, et le produit méthylolé est un alcool phénylméthylique répondant à la formule:

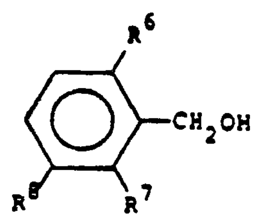

dans laquelle $R^6$, $R^7$ et $R^8$ sont tels que définis ci-dessus.

15. Procédé de la revendication 14, caractérisé en ce que $R^6$ est un atome d'hydrogène ou un groupe méthyle, $R^7$ est un groupe méthyle et $R^8$ est un atome de chlore ou un groupe phényle, sous réserve que lorsque $R^8$ est un atome de chlore, $R^6$ est un atome d'hydrogène.

16. Procédé de la revendication 15, caractérisé en ce que $R^6$ est un atome d'hydrogène.

17. Procédé de la revendication 16, caractérisé en ce que $R^8$ est un groupe phényle.

18. Procédé de la revendication 17 qui est effectué dans des conditions pratiquement anhydres, en présence d'une atmosphère pratiquement inerte, à une température de 25° C, jusqu'à une température inférieure au point de fusion du polymère du formaldéhyde.

19. Procédé de la revendication 18, caractérisé en ce que la température est dans l'intervalle de 60 à 100° C.

20. Procédé de la revendication 19, caractérisé en ce que le polymère du formaldéhyde a une masse moléculaire moyenne dans l'intervalle de 15 000 à 150 000.

21. Procédé de la revendication 20, caractérisé en ce que la masse moléculaire moyenne du polymère du formaldéhyde est dans l'intervalle de 20 000 à 100 000.

22. Procédé de la revendication 21, caractérisé en ce que la masse moléculaire moyenne du polymère du formaldéhyde est dans l'intervalle de 25 000 à 75 000.